**Europäisches Patentamt**

**European Patent Office**

**Office européen des brevets**

(19)

(11) Veröffentlichungsnummer: **0 101 981**
**B1**

# EUROPÄISCHE PATENTSCHRIFT

(45) Veröffentlichungstag der Patentschrift:
06.04.88

(21) Anmeldenummer: 83107701.1

(22) Anmeldetag: 03.08.83

(51) Int. Cl.⁴: **A 61 B 10/00,** A 61 F 13/20

(54) **Verpackungsröhrchen.**

(30) Priorität: 23.08.82 DE 3231314

(43) Veröffentlichungstag der Anmeldung:
07.03.84 Patentblatt 84/10

(45) Bekanntmachung des Hinweises auf die Patenterteilung:
06.04.88 Patentblatt 88/14

(84) Benannte Vertragsstaaten:
AT BE CH FR GB IT LI LU NL SE

(56) Entgegenhaltungen:
DE-C-938 034
FR-A-2 216 975
GB-A-1 162 249
US-A-4 184 483

(73) Patentinhaber: **Medi- Pharma Vertriebsgesellschaft mbH, Sachsenring 37- 47, D-5000 Köln 1 (DE)**

(72) Erfinder: **Roy, Asok Kumar, Dr., Strünckweg 11, D-1000 Berlin 13 (DE)**
Erfinder: **Klinke, Horst- Dieter, Beatestrasse 9b, D-1000 Berlin 13 (DE)**

(74) Vertreter: **Groening, Hans Wilhelm, Dipl.- Ing., Patentanwälte Strehl Schübel- Hopf Groening Schulz Widenmayerstrasse 17 Postfach 22 03 45, D-8000 München 22 (DE)**

EP 0 101 981 B1

LIBER, STOCKHOLM 1988

## Beschreibung

Die Erfindung betrifft ein Verpackungsröhrchen aus Metall hoher Wärmeleitfähigkeit für einen aus zwei teleskopartig ineinander verschiebbaren Hülsen, einer Einführhülse und einer Ausstoßhülse, bestehenden Tamponapplikator für die humanmedizinische Diagnose von pathologischen Veränderungen in weiblichen Körperhöhlen, vorzugsweise in der Vagina, mit einem Verschlußstopfen aus elastischem Material und einem Außendurchmesser, der in dem an die Öffnung des Verpackungsröhrchens anschließenden Längsabschnitt kleiner bemessen ist als ein Längenabschnitt mit konischem Übergang zu einem Längenabschnitt größeren Durchmessers, und einem Öffnungsrand, dessen Außendurchmesser kleiner bemessen ist als in den Längenabschnitten größeren Durchmessers, wobei der Verschlußstopfen an seiner mit der Innenfläche des Röhrchens in Berührung kommenden Umfangsfläche in einem Abstand von etwa einem Viertel seiner Länge, ausgehend von dem dem Verpackungsröhrchen zugewandten Ende, mit mindestens einem Vorsprung versehen ist und am Umfang seines dem Verpackungsröhrchen zugewandten Endes mindestens eine Ausnehmung aufweist.

Ein Verpackungsröhrchen dieser Gattung ist aus der GB-A-1 162 249 bekannt. Aus dieser Druckschrift sind Stopfen für Behälter aus Metall hoher Wärmeleitfähigkeit zum Gefriertrocknen einer darin enthaltenen Substanz bekannt, deren schaftähnlicher Teil aus einem Befestigungsbereich besteht, der in dem Hals des Behälters zur Anlage kommt, ferner aus einem Dichtungsbereich, der mindestens zum Teil im wesentlichen zylindrisch ausgebildet ist, sowie einem dazwischenliegenden Zwischenbereich und Durchtrittskanälen, die in das Innere des Behälters führen, wenn der Verschlußstopfen in den Behälter teilweise eingesetzt ist und sich aus dem Zwischenbereich heraus erstreckt. Behälter dieser Art dienen zum Trocknen von insbesondere pharmazeutischen Produkten, die durch Vakuumverdampfung bei geringer Temperatur getrocknet und pulverisiert werden. Auf diese Weise wird die Lagerfähigkeit derartiger Substanzen ohne Beeinträchtigung ihrer Wirksamkeit beträchtlich erhöht. Das Gefriertrocknungsverfahren wird in der Weise durchgeführt, daß der Verschlußstopfen mit seinem Befestigungsbereich in den Hals des Behälters eingesetzt wird. Die in das Innere des Behälters führenden Kanäle erstrecken sich dabei über den Behälterhals hinaus und stellen eine Verbindung mit der Außenseite her. Die Behälter werden in diesem Zustand in einen verschließbaren Gefriertrockner eingesetzt, in dessen Innern die Bedingungen herrschen, die zum Gefriertrocknen, wie verminderter Druck und Temperatur, nötig sind. Auf diese Weise wird der Substanz durch die Kanäle hindurch der Wassergehalt entzogen. Nach der Beendigung des Verfahrens kann eine Verschlußplatte innerhalb des geschlossenen Gefriertrockners abgesenkt werden, so daß die Verschlußstopfen bis zu ihrem Dichtungsbereich in den Behälter hineingepreßt werden können, so daß diese vollständig abgedichtet sind. Der Verschlußstopfen ist zwischen seinem Befestigungsbereich und dem Zwischenbereich mit radial vorstehenden elastischen Anschlagorganen versehen, die beim Einsetzen des Verschlußstopfens in den Behälter dazu dienen, den Stopfen darin festzuhalten, bis eine zusätzliche Kraft zur Überwindung der Elastizität der radialen Vorsprunge ausgeübt und der Verschlußstopfen in den Behälter zur vollständigen Abdichtung hineingedrückt wird. Der Außendurchmesser des Öffnungsrandes ist kleiner als der Längenabschnitt größeren Durchmessers, so daß die Behälter ohne gegenseitige Behinderung dicht nebeneinander angeordnet werden können. Die Höhe des Längenabschnittes kleineren Durchmessers des Behälters beschränkt sich auf die Höhe des Verschlußstopfens.

Es sind auch Tampons bekannt, die z. B. für die Frauenhygiene oder für medizinische Zwecke vorgesehen sind. Darunter befinden sich spezielle, z. B. aus Baumwoll- und/oder Zellulosefasern bestehende Tampons, die für Diagnose- oder Therapiezwecke mit einem oder mehreren chemischen Wirkstoffen beschichtet oder getränkt sind.

Da diese Wirkstoffe häufig, z. B. bei Diagnosen, auf sehr kleine stoffliche Veränderungen, beispielsweise in einer menschlichen Körperhöhle, reagieren müssen, sind sie oft selbst sehr empfindliche Stoffe. Es ist deshalb erforderlich, diese Stoffe bzw. die damit versehenen Trägermaterialien, z. B. mit einem Diagnosemittel getränkte Tampons, vom Zeitpunkt ihrer Herstellung bis zu ihrer Verwendung vor schädlichen Umwelteinflüssen, wie Luftsauerstoff, Luftfeuchtigkeit und energiereichem Licht, zu schützen. Dabei ist unter "energiereichem Licht" solches Licht (einschließlich UV-Licht) zu verstehen, das energiereicher als Rotlicht ist. Diesen Erfordernissen muß daher die für den Tampon gewählte Verpackungshülle genügen.

Da diagnostische und therapeutische Tampons bis zu ihrer Anwendung besonders sauber gehalten werden müssen, um keine störenden Nebenreaktionen auszulösen, werden die Tampons meist schon bei oder unmittelbar nach ihrer Herstellung in Tamponapplikatoren eingelegt. Diese ermöglichen ein Einführen eines Tampons in eine Körperhöhle, ohne den Tampon mit den Fingern zu verunreinigen.

Der Erfindung liegt die Aufgabe zugrunde, ein Verpackungsröhrchen der vorgenannten bekannten Gattung für einen Tampon für die medizinische Diagnose von pathologischen Veränderungen bei Menschen und Tieren, zum Beispiel in weiblichen Körperhöhlen, vorzugsweise in der Vagina, anzugeben. Dabei soll das Verpackungsröhrchen eine axiale Ausrichtung des Applikators in dem Verpackungsröhrchen, das heißt, eine genaue Positionierung des den Tampon enthaltenden Applikators ermöglichen, um für das Aufbringen einer Wirkstofflösung eine größere Sicherheit und gleichzeitig eine über den ganzen Umfang des Tampons gleichmäßige Kühlung zu erzielen.

Die Erfindung löst diese Aufgabe durch die im Kennzeichen des Patentanspruchs 1 enthaltenen Merkmale.

Durch den gegenüber der Eindringtiefe des Verschlußstopfens längeren Bereich des verengten zylindrischen Bereichs des Verpackungsröhrchens wird ein in das Verpackungsröhrchon eingebrachter Tamponapplikator, der zum Beispiel aus einer Einführhülse und einer darin teleskopartig verschiebbaren Ausstoßhülse besteht, im Bereich der Öffnung der Verpackungshülse zentriert, was zusammen mit der Zentrierung des anderen Endes eines derartigen Tamponapplikators am Boden des Verpackungsröhrchens eine zu diesem etwa koaxiale Ausrichtung ermöglicht. Dadurch wird die Betriebssicherheit wesentlich erhöht, wenn mittels einer automatischen Vorrichtung eine Wirkstofflösung auf das Einführende eines Tampons aufgespritzt wird, der in dem Applikator in dem Verpackungsröhrchen angeordnet ist. Da außerdem der Tampon über seinen ganzen Umfang durch die axiale Ausrichtung etwa einen gleichen Abstand von der Innenseite des Verpackungsröhrchens einnimmt, ist auch eine gleichmäßige Kühlung und ein gleichmäßiger Wasserentzug gewährleistet. Die Zentriervorrichtung am Boden besteht vorzugsweise aus einer dem Verpackungsgut angepaßten Vertiefung im Boden des Verpackungsröhrchens.

Der verringerte Außendurchmesser bewirkt auch, daß ein in das Verpackungsröhrchen eingebrachter Tamponapplikator, der z. B. aus einer Einführhülse und einer darin teleskopartig verschiebbaren Ausstoßhülse besteht, im Bereich der Öffnung der Verpackungshülse zentriert wird, d.h. einen nur geringen radialen Spielraum gegenüber der Innenwand des Röhrchens hat. Dadurch wird die Betriebssicherheit wesentlich erhöht, wenn mittels einer automatischen Vorrichtung eine Wirkstofflösung auf das Einführende eines Tampons aufgespritzt wird, der in dem Applikator im Verpackungsröhrchen angeordnet ist.

Nachfolgend ist die Erfindung anhand von in der Zeichnung schematisch dargestellten Ausführungsbeispielen erläutert.

Es zeigen:

Fig. 1 Teile eines vergrößerten Längsschnittes durch eine erste Ausführungsform des Verpackungsröhrchens;

Fig. 2 eine Vergrößerung des Ausschnitts A in Fig. 1;

Fig. 3 eine Vergrößerung des Ausschnitts B in Fig. 1;

Fig. 4 eine vergrößerte Seitenansicht eines Verschlußstopfens für das Verpackungsröhrchen gemäß Fig. 1;

Fig. 5 eine vergrößerte Ansicht der Unterseite des Verschlußstopfens gemäß Fig. 4;

Fig. 6 einen Längsschnitt durch ein Verpackungsröhrchen gemäß Fig. 1 sowie durch einen darin angeordneten Tamponapplikator mit Tampon, wobei der Verschlußstopfen nur teilweise aufgesetzt und in Seitenansicht gezeigt ist;

Fig. 7 einen Längsschnitt ähnlich Fig. 6 einschließlich eines vollständig aufgesetzten Verschlußstopfens und mit einer Vertiefung im Boden des Verpackungsröhrchens.

Ein Verpackungsröhrchen 1, das z. B. aus Aluminium besteht, weist einen Boden 2, eine Wandung 3 mit Längenabschnitten 3a, 3b, 3c sowie eine Öffnung 4 mit einem nach außen umgebördelten Rand 5 auf. Der Querschnitt des Verpackungsröhrchens 1 ist über seine gesamte Länge kreiszylindrisch.

Im Längenabschnitt 3a ist der Außendurchmesser des Verpackungsröhrchens 1 größer als im Längenabschnitt 3c und am Bördelrand 5. Im Längenabschnitt 3b vermindert sich der Außendurchmesser des Verpackungsröhrchens 1 konisch nach oben. Insgesamt ergibt sich dadurch eine flaschenähnliche Form des Verpackungsröhrchens 1.

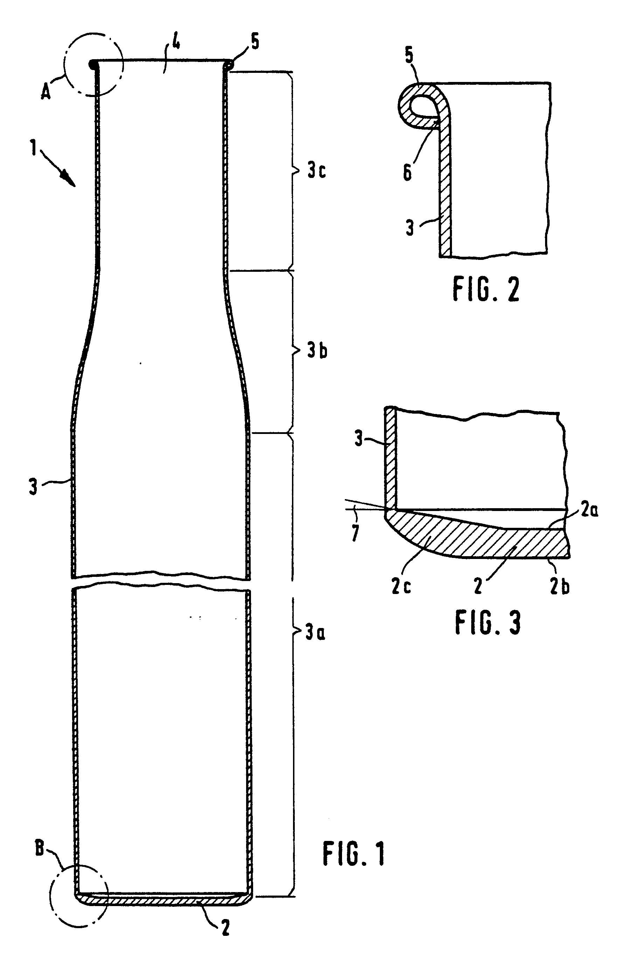

Der Ausschnitt A in Fig. 1 ist in Fig. 2 vergrößert dargestellt und zeigt, daß der Rand 5 derart nach außen umgebördelt ist, daß er im Querschnitt etwa kreisförmig gekrümmt ist und die Schnittkante 6 der Wandung 3 an deren Außenseite anliegt. Dadurch ist ein Verletzungsrisiko durch die Schnittkante 6 ausgeschlossen.

Der Ausschnitt B in Fig. 1 ist in Fig. 3 vergrößert gezeigt. Daraus ist ersichtlich, daß der Boden 2 dicker bemessen ist als die Wandung 3. Außerdem sind die Innenfläche 2a und die Außenfläche 2b des Bodens 2 größtenteils senkrecht zur Wandung 3 angeordnet. Lediglich in einem der Wandung 3 unmittelbar benachbarten Bodenabschnitt 2c steigt die Innenfläche 2a konisch gegen die Wandung 3 an, während die Außenfläche 2b durch Abrundung in die Wandung 3 übergeht.

Die im Bodenabschnitt 2c konisch verlaufende Innenfläche 2a bildet mit der Horizontalen einen Winkel 7 von etwa 10°.

Die Länge des Abschnitts 3a beträgt z. B. 110 mm, die Länge des Abschnitts 3b z. B. 10 mm und die Länge des Abschnitts 3c z. B. 26 mm. Die Dicke des Bodens 1 liegt z. B. bei 0,6 bis 1 mm, die Dicke der Wandung 3 im Längenabschnitt 3a z. B. bei 0,23 mm und im Längenabschnitt 3c bei z. B. 0,28 mm.

Für den Außendurchmesser im Längenabschnitt 3a sind z. B. 22 mm, für den Außendurchmesser im Längenabschnitt 3c z. B. 16 mm und für den Außendurchmesser am Bördelrand 5 z. B. 18 mm geeignet. Die Höhe des Bördelrandes 5 kann z. B. 1,2 mm betragen.

Die vorgenannten Abmessungen können aber je nach dem Verwendungszweck des Verpackungsröhrchens und der Art seines Materials sowie in Abhängigkeit von der Art und der Gestalt des zu verpackenden Gegenstands über- oder unterschritten werden.

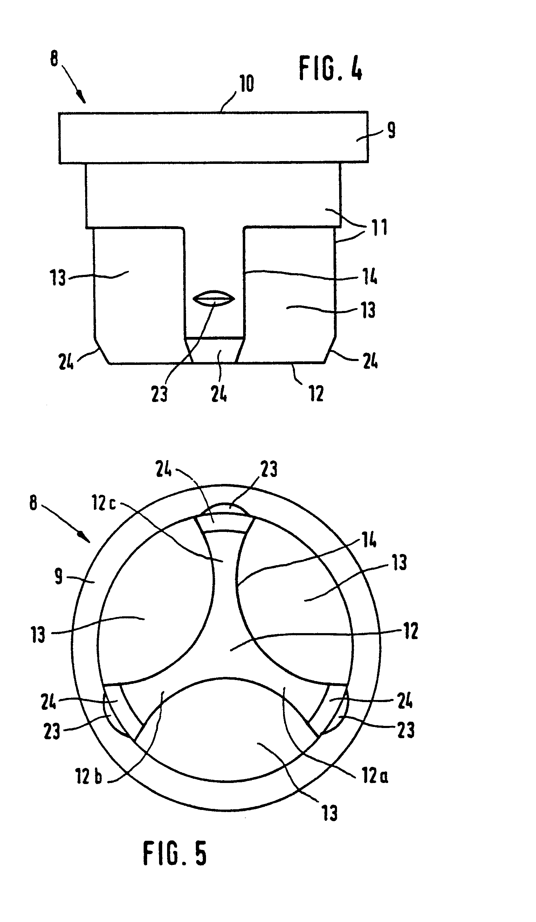

Zum Verschließen der Öffnung 4 ist ein Verschlußstopfen 8 aus einem elastischen Material, z. B. Weichgummi, vorgesehen. Der in Fig. 4 dargestellte Verschlußstopfen 8 weist einen scheibenartigen Griffteil 9 mit einer äußeren Stirnfläche 10 auf. Der Durchmesser des Griffteils 9 ist größer als der Außendurchmesser des darunter befindlichen Dichtungsteils 11 mit einer inneren Stirnfläche 12.

An drei der Stirnfläche 12 benachbarten, symmetrisch verteilten Umfangsabschnitten des Dichtungsteils 11 sind drei Ausnehmungen 13 vorgesehen. Aus Fig. 5, die eine Draufsicht auf die innere Stirnfläche 12 zeigt, ist ersichtlich daß die Begrenzungsfläche einer jeden Ausnehmung 13 bezüglich der Achse des Verschlußstopfens konvex gekrümmt ist. Außerdem zeigen die Fig. 4 und 5, daß die Begrenzungsflächen 14 parallel zur Achse des Verschlußstopfens 8 verlaufen. Durch diese Gestaltung ist der dem Verpackungsröhrchen 1 zugewandte Teil des Verschlußstopfens 8, der sich über etwa die halbe Stopfenlänge erstreckt, in Form von drei Rippen ausgebildet.

Die Ausnehmungen 13 reichen so weit an die Achse des Verschlußstopfens 8, daß die innere Stirnfläche 12 im wesentlichen nur noch von drei schmalen Flächenabschnitten 12a, 12b, 12c gebildet wird, die sich an der Achse des Verschlußstopfens 8 überlappen und von dort radial nach außen erstrecken, wobei die Längenachsen von zwei benachbarten Flächenabschnitten 12a, 12b, 12c einen Winkel von 120° bilden.

Am Umfang des Dichtungsteils 11 sind in Höhe der Ausnehmungen 13 Vorsprünge 23 vorgesehen, die gegenüber dem Bördelrand 5 als Anschläge dienen, wenn der Verschlußstopfen 8 nur teilweise in das Verpackungsröhrchen 1 eingedrückt werden soll.

Der Übergang zwischen der Umfangsfläche des Dichtungsteils 11 und der Stirnfläche 12 ist unter Ausbildung von Randflächen 24 konisch abgeschrägt, um das Einsetzen des Verschlußstopfens 8 in das Verpackungsröhrchen 1 zu erleichtern.

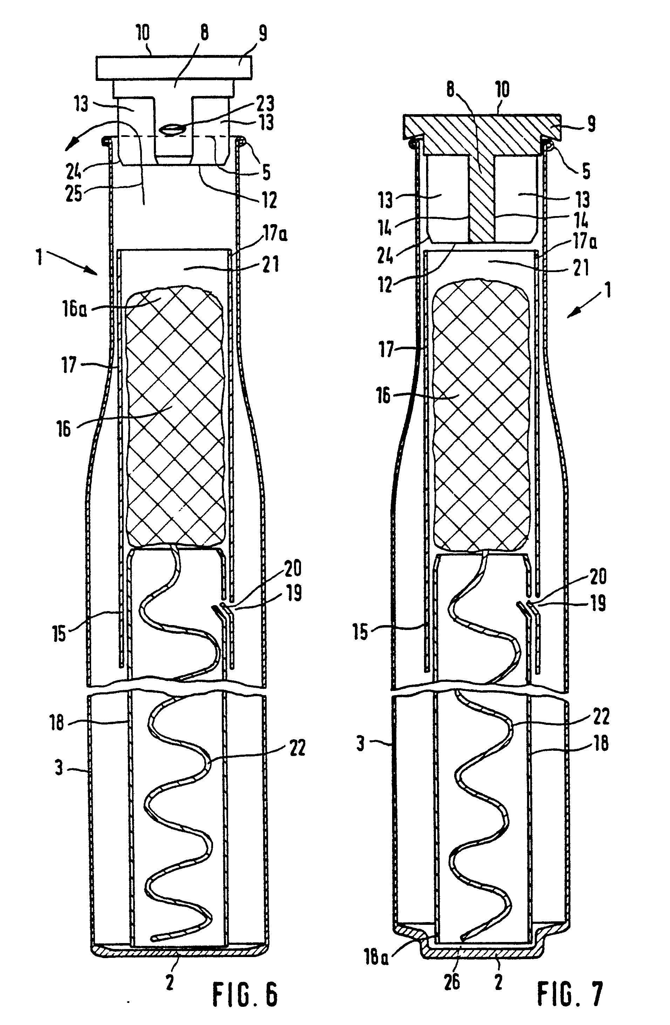

In den Fig. 6 und 7 ist dargestellt, wie ein Tamponapplikator 15, in dem sich ein Tampon 16 befindet, in dem Verpackungsröhrchen 1 angeordnet ist.

Der Applikator 15 besteht aus einer Einführhülse 17 und einer darin teleskopartig eingeschobenen Ausstoßhülse 18 mit etwas kleinerem Durchmesser. In dem Bereich, in dem sich die Einführhülse 17 und die Ausstoßhülse 18 überlappen, sind beide Hülsen 17,18 mit einer Einstanzung 19 versehen. Dadurch wird in der Wandung der Einführhülse 17 eine Zunge 20 gebildet, die in die Einstanzung 19 der Ausstoßhülse 18 hineinragt und so ein unbeabsichtigtes Herausgleiten der Ausstoßhülse 18 aus der Einführhülse 17 verhindert.

Diese Arretierung kann aber auch in anderer Weise verwirklicht sein, z. B. durch eine Umfangsnut an der Ausstoßhülse 18 und einen Innenwulst an der Einführhülse 17 bzw. umgekehrt. Es liegt dann eine gegenseitige Fixierung der beiden Hülsen 17, 18 ähnlich einer Rastverbindung vor.

Der Tampon 18 ist so in der Einführhülse 17 angeordnet, daß das Einführende 17a der Einführhülse 17 das Einführende 18a des Tampons 16 überragt und dabei einen freien Raum 21 am Einführende 17a der Einführhülse 17 bildet. Dieser freie Raum 21 dient dazu, beim Aufbringen eines Wirkstoffs auf das Einführende 16a des Tampons 18 die dorthin rasch aufgespritzte Wirkstofflösung aufzunehmen, die eine etwas längere Zeit benötigt, um in den Tampon 16 einzudringen. Der Raum 21 faßt etwa 3 ml Lösung.

Um zu verhindern, daß während des Vorliegens der genannten Lösung in dem Raum 21 und auch später ein Teil der Lösung oder des Wirkstoffs in die Wandung der Einführhülse 17 abwandert und dadurch für den Tampon verloren geht, kann die Innenwand der Einführhülse 17 mit einem undurchlässigen Material, z. B. mit einem Silicon, einem Wachs, einer Kunststoffschicht bzw. -folie, wie einer PVDC-Folie, oder einer Aluminiumfolie, beschichtet sein.

Das Rückholband 22 des Tampons 16 erstreckt sich durch die Ausstoßhülse 18.

In der Fig. 6 ist der Verschlußstopfen 8 nicht in einem Längsschnitt, sondern in einer Seitenansicht gezeigt, um die Wirkungsweise der Vorsprünge 23 zu erläutern. Wenn nach dem Aufbringen einer Wirkstofflösung auf den Tampon 16 erwünscht ist, den Verschlußstopfen 8 zunächst nur teilweise in die Öffnung 4 des Verpackungsröhrchens 1 einzudrücken, fungieren die Vorsprünge 23 als Anschläge gegenüber dem Bördelrand 5, der in Fig. 6 als gestrichelte Linie dargestellt ist. In dieser Position des Verschlußstopfens 8 bleibt mittels der Ausnehmungen 13 ein Strömungskanal 25 zwischen dem Innenraum des Verpackungsröhrchens 1 und seiner äußeren Umgebung frei. Dies ist während der Behandlung des Verpackungsröhrchens samt Inhalt in der Gefriertrocknungsvorrichtung erforderlich. Nach Beendigung des Trocknungsvorgangs kann der Verschlußstopfen 8 bis zum Anschlag des Griffteils 9 an den Bördelrand 5 in das Verpackungsröhrchen 1 eingedrückt werden.

Fig. 7 entspricht weitgehend Fig. 6. Jedoch ist in Fig. 7 der Verschlußstopfen 8 in der Verschlußstellung und im Längsschnitt gezeigt.

Außerdem weist das Verpackungsröhrchen 1 gemäß Fig. 7 eine Vertiefung 26 im Boden 2 auf, deren Durchmesser etwas größer ist als der Außendurchmesser des darin angeordneten rückwärtigen Endes 18a der Ausstoßhülse 18. Auf diese Weise wird der Tamponapplikator 15 an seinem rückwärtigen Ende im Verpackungsröhrchen 1 zentriert und so einerseits gegen radiales Verrutschen an diesem Ende geschützt sowie andererseits axial zu einer nicht dargestellten Injektionsnadel ausgerichtet, mittels der eine Wirkstofflösung auf den Tampon 16 aufgespritzt wird.

Bei der Verwendung des Verpackungsröhrchens 1 kann z. B. wie folgt vorgegangen werden:

In das aufrechtstehende Verpackungsröhrchen 1 wird der den Tampon 16 enthaltende Applikator 15 derart eingelegt, daß das Einführende des Tampons der Öffnung 4 des Verpackungsröhrchens 1 zugewandt ist. Mit einer automatisch arbeitenden Einfüllvorrichtung werden dann mittels einer Injektionsnadel etwa 2 ml einer Wirkstofflösung in den freien Raum 21 eingespritzt. Anschließend wird der Verschlußstopfen 8 teilweise in die Öffnung 4 des Verpackungsröhrchens 1 eingedrückt, wie es in Fig. 6 dargestellt ist. Eine Vielzahl derart vorbereiteter Verpackungsröhrchen 1, die unter gegenseitiger Berührung dicht nebeneinander gestellt sind, wird in eine Gefriertrocknungsvorrichtung eingebracht. Durch die gegenseitige Berührung der Verpackungsröhrchen 1 ist ein schnelles Abkühlen der Röhrchen und ihres Inhalts gewährleistet. Durch das angelegte Vakuum verdampft das Lösungsmittel, z. B. Wasser, der Wirkstofflösung aus dem Tampon 16 und tritt über die Ausnehmungen 13 im Verschlußstopfen 8 aus dem Verpackungsröhrchen 1. Nach Beendigung des Trocknungsvorgangs werden die Verschlußstopfen 8 jeweils bis zum dichtenden Verschluß in die Verpackungsröhrchen 1 eingedrückt.

Insgesamt gesehen erleichtert das Verpackungsröhrchen 1 das Aufbringen eines Wirkstoffs auf den Tampon 16, macht das Trocknen des Röhrcheninhalts kostengünstiger und gewährleistet durch Ausschluß von Feuchtigkeit, Luft und Licht eine lange Haltbarkeit des verpackten Tampons 16 bis zum Zeitpunkt seiner Verwendung für Diagnose- oder Therapiezwecke.

**Patentansprüche**

1. Verpackungsröhrchen (1) aus Metall hoher Wärmeleitfähigkeit für einen aus zwei teleskopartig ineinander verschiebbaren Hülsen, einer Einführhülse (17) und einer Ausstoßhülse (18), bestehenden Tamponapplikator (15) für die humanmedizinische Diagnose von pathologischen Veränderungen in weiblichen Körperhöhlen, vorzugsweise in der Vagina,
mit einem Verschlußstopfen (8) aus elastischem Material,
und einem Außendurchmesser, der in dem an die Öffnung (4) des Verpackungsröhrchens anschließenden Längenabschnitt (3c) kleiner bemessen ist als ein Längenabschnitt (3b) mit konischem Übergang zu einem Längenabschnitt (3a) größeren Durchmessers,
und einem Öffnungsrand (5), dessen Außendurchmesser kleiner bemessen ist als in den Längenabschnitten (3a, 3b) größeren Durchmessers, wobei der Verschlußstopfen an seiner mit der Innenfläche des Röhrchens in Berührung kommenden Umfangsfläche in einem Abstand von etwa einem Viertel seiner Länge, ausgehend von dem dem Verpackungsröhrchen zugewandten Ende mit mindestens einem Vorsprung (23) versehen ist und am Umfang seines dem Verpackungsröhrchen zugewandten Endes mindestens eine Ausnehmung (13) aufweist,
dadurch gekennzeichnet,
daß der Innendurchmesser des sich an die Öffnung (4) anschließenden Längenabschnitts (3c) geringfügig größer als der Außendurchmesser des Einführendes (17a) der Einführhülse (17) des Tamponapplikators bemessen ist, daß der sich an die Öffnung (4) anschließende Längenabschnitt (3c) länger als die Eindringtiefe des Verschlußstopfens bemessen ist,
daß der Abstand zwischen der inneren Stirnfläche (12) des Verschlußstopfens und dem Boden (2) des Verpackungsröhrchens etwas größer bemessen ist als die Länge des Tamponapplikators,
wobei der Tamponapplikator im Bereich der Öffnung des Verpackungsröhrchens zentriert wird, und
daß eine Zentriervorrichtung für den Tamponapplikator am Boden (2) des Verpackungsröhrchens vorgesehen ist.

2. Verpackungsröhrchen (1) nach Anspruch 1, dadurch gekennzeichnet, daß die Zentriervorrichtung aus einer Vertiefung (26) im Boden (2) besteht, deren Durchmesser etwas größer bemessen ist als der Außendurchmesser des rückwärtigen Endes (18a) der Ausstoßhülse (18) des Tamponapplikators (15).

**Claims**

1. Packaging tubule (1) of metal of high thermal conductivity for a tampon applicator (15), which consists of two sleeves telescopically displaceable in one another, namely an introduction sleeve (17) and an ejection sleeve (18), for the diagnosis, in human medicine, of pathological changes in female body cavities, preferably in the vagina, having a closing plug (8) of elastic material, and and external diameter which, in the length section (3c) adjoining the opening (4) of the packing tubule is smaller than in a length section (3b) with a conical transition to a length section (3a) of larger diameter, and an opening edge (5), the external diameter of which is smaller than that in the length sections (3a, 3b) of larger diameter, the closing plug being provided with at least one projection (23) on its peripheral surface which comes into contact with the internal surface of the tubule, at a distance of about one quarter of its length starting from the end facing the packaging tubule, and having at least one recess (13) on the periphery of its end facing the opening of the packaging tubule, characterized in that the internal diameter of the length section (3c) adjoining the opening (4) is slightly larger than the external diameter of the introduction end (17a) of the introduction sleeve (17) of the tampon applicator,

the length section (3c) adjoining the opening (4) is longer than the depth of penetration of the closing plug, the distance between the inner end face (12) of the closing plug and the bottom (2) of the packaging tubule is slightly larger than the length of the tampon applicator, the tampon applicator being centered in the region of the opening of the packaging tubule, and

a centering device for the tampon applicator is provided in the bottom (2) of the packaging tubule.

2. Packaging tubule (1) as claimed in claim 1, characterized in that the centering device consists of a depression (26) in the bottom (2), the diameter of which is slightly larger than the external diameter of the rear end (18a) of the ejection sleeve (18) of the tampon applicator (15).


**Revendications**

1. Petit tube d'emballage (1) en métal à haute conductibilité thermique pour un tampon applicateur (15) constitué par deux gaines aptes à coulisser télescopiquement l'une dans l'autre, une gaine d'introduction (17) et une gaine d'éjection (18), pour le diagnostic en médecine humaine de modifications pathologiques des cavités corporelles de la femme, de préférence du vagin, muni d'un bouchon d'obturation (8) en matériau élastique,

et ayant un diamètre extérieur qui est dimensionné de façon à être plus petit dans la section longitudinale (3c) se raccordant sur l'ouverture (4) du petit tube d'emballage qu'une section longitudinale (3b) ayant une transition conique sur une section longitudinale (3a) de plus grand diamètre,

et une bordure d'ouverture (5) dont le diamètre extérieur est dimensionné de façon à être plus petit que dans les sections longitudinales (3a, 3b) de plus grand diamètre, le bouchon d'obturation étant muni d'au moins une saillie (23) sur sa face périphérique venant en contact avec la face intérieure du petit tube à une distance d'environ un quart de sa longueur, en partant de l'éxtrémité oppose'e au petit tube d'emballage, et présentant au moins un évidement (13) sur la périphérie de son extrémité opposée au petit tube d'emballage,

caractérisé en ce que:

le diamètre intérieur de la section longitudinale (3c) se raccordant sur l'ouverture (4) est dimensionné de façon à être légèrement plus grande que le diamètre extérieur de l'extrémité d'introduction (17a) de la gaine d'introduction (17) du tampon applicateur, en ce que la section longitudinale (3c) se raccordant sur l'ouverture (4) est dimensionnée de façon à être plus longue que la profondeur de pénétration du bouchon d'obturation, en ce que la distance entre la face frontale interne (12) du bouchon d'obturation et le fond (2) du petit tube d'emballage est dimensionnée de façon à être sensiblement plus grande que la longueur du tampon applicateur,

le tampon applicateur étant centré dans la zone d'ouverture du petit tube d'emballage, et

en ce que le dispositif de centrage pour le tampon applicateur est prévu sur le fond (2) du petit tube d'emballage.

2. Petit tube d'emballage (1) selon la revendication 1, caractérisé en ce que le dispositif de centrage est constitué par une dépression (26) dans le fond (2), dont le diamètre est dimensionné de façon à être sensiblement plus grand que le diamètre extérieur de l'extrémité arrière (18a) de la gaine d'éjection (18) du tampon applicateur (15).

FIG. 2

FIG. 3

FIG. 1

FIG. 4

FIG. 5

FIG. 6    FIG. 7